(19) Europäisches Patentamt
European Patent Office
Office européen des brevets

(11) Publication number: **0 471 296 B1**

(12) **EUROPEAN PATENT SPECIFICATION**

(45) Date of publication of patent specification: **08.11.95**

(51) Int. Cl.[6]: **C07D 401/06**, A61K 31/44

(21) Application number: **91113333.8**

(22) Date of filing: **08.08.91**

(54) **1-(Pyridinylalkyl)-1H-indoles, indolines and related analogs.**

(30) Priority: **13.08.90 US 566575**

(43) Date of publication of application:
**19.02.92 Bulletin 92/08**

(45) Publication of the grant of the patent:
**08.11.95 Bulletin 95/45**

(84) Designated Contracting States:
**AT BE CH DE DK ES FR GB GR IT LI LU NL SE**

(56) References cited:

**CHEMICAL ABSTRACTS, vol. 70, no. 5, February 3, 1969, Columbus, Ohio, USA SHEINKMAN, A.K. et al. "Synthesis and pharmacology of N-(pyridylalkyl)-indolines and -indoles." page 1973, column 2, abstract-no. 19 857b & Khim.-Farm. Zh. 1968, 2(9), 29-35**

**CHEMICAL ABSTRACTS, vol. 97, no. 7, August 16, 1982, Columbus, Ohio, USA BOSCH, JOAN et al. "Model studies in the vinoxine series." page 679, column 2, abstract-no. 56 098h & Heterocycles 1982, 19(5), 853-6**

(73) Proprietor: **HOECHST-ROUSSEL PHARMACEUTICALS INCORPORATED**
**Route 202-206 North**
**Somerville**
**New Jersey 08876 (US)**

(72) Inventor: **Effland, Richard Charles**
**544 Rolling Hills Road**
**Bridgewater, NJ 08807 (US)**
Inventor: **Davis, Larry**
**P.O. 129,**
**Bird Lane**
**Sergeantsville, NJ 08557 (US)**
Inventor: **Olsen, Gordon E.**
**8K Franklin Greens**
**Somerset, NJ 088673 (US)**

(74) Representative: **Isenbruck, Günter, Dr. et al**
**HOECHST AKTIENGESELLSCHAFT**
**Zentrale Patentabteilung**
**Gebäude F 821**
**D-65926 Frankfurt am Main (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 471 296 B1

CHEMICAL ABSTRACTS, vol. 100, no. 3, January 16, 1984, Columbus, Ohio, USA BOSCH, JOAN et al. "Mercuric acetate cyclization of 4-(pyrrolylmethyl)-and 4-(indolylmethyl)piperidines to bridget polycyclic systems". page 484, column 1, abstract-no. 22 552s & J. Org. Chem. 1983, 48(25), 4836-41

CHEMICAL ABSTRACTS, vol. 105, no. 11, September 15, 1986, Columbus, Ohio, USA BOSCH, JOAN et al. "Synthetic studies on the indole alkaloid vinoxine. Synthesis of 19,20-dihydro-16-epivinoxine." page 666, column 1, abstract-no. 97 774c & J. Org. Chem. 1986, 51(12), 2289-97

CHEMICAL ABSTRACTS, vol. 106, no 21, May 25, 1987, Columbus, Ohio, USA BENNASAR, M. LLUISA et al. "Synthetic applications of 2-cyanopiperidines. II. Model studies in the synthesis of the indole alkaloid vinoxine."page 762, column 1, abstract-no. 176 716j & Tetrahedron 1986, 42(2), 637-47

CHEMICAL ABSTRACTS, vol. 109, no. 17, October 14, 1988, Columbus, Ohio, USA ALY, MOUSTAFA F. et al. "The reaction of secondary alpha-amino acids with carbonyl compounds. Properties of the intermediate azomethineylides. Oxazolidine formation versus 1,4-prototropy." page 784, column 1, abstract-no. 150 001j & Tetrahedron Lett. 1987, 28(48), 6077-80

CHEMICAL ABSTRACTS, vol. 82, no. 9, March 3, 1975, Columbus, Ohio, USA WHITEHEAD, CALVERT W. et al. "Effect of lipophilic substituents on some biological properties of indoles." page 17, column 1, abstract-no. 51 350q & J. Med. Chem. 1974, 17(12), 1998-304

## Description

This invention relates to compounds of the formula

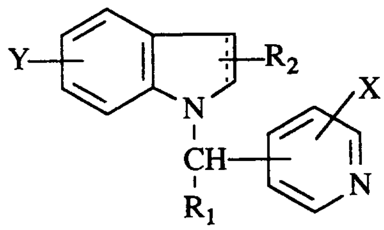

(I)

wherein

- $R_1$ is hydrogen, $C_1$-$C_6$-alkyl, phenyl-$C_1$-$C_6$-alkyl, $C_2$-$C_6$-alkenyl or $C_2$-$C_6$-alkynyl;
- $R_2$ is hydrogen;
- X is hydrogen, halogen, nitro, amino, $C_1$-$C_6$-alkyl, $C_1$-$C_6$-alkoxy or hydroxy;
- Y is hydrogen, $C_1$-$C_6$-alkyl, $C_1$-$C_6$-alkoxy, phenyl-$C_1$-$C_6$-alkoxy, hydroxy, halogen, nitro or amino; the dotted line in formula I signifies an optional bond; and wherein the phenyl group in each occurence may be mono- or disubstituted with $C_1$-$C_6$-alkyl, $C_1$-$C_6$-alkoxy, halogen or trifluoromethyl, and with the proviso, that at least one of the substituents $R_1$ and Y is not hydrogen; the pharmaceutically acceptable acid addition salts thereof, and, where applicable, the geometric and optical isomers and racemic mixtures thereof. The compounds of the invention are useful for the treatment of various memory dysfunctions characterized by a decreased cholinergic function, such as Alzheimer's disease.

In C. A. 1969 70: 19857b; J. Org. Chem. 1986, 51, 2289; Tetrahedron 1986, 42, 637; C. A. 1988, 109: 150001j; and J. Med. Chem. 1974 17, 1298 there have been disclosed 1-(2,3, or 4-pyridylmethyl)-indoles and -indolines, respectively, which may also be substituted at the pyridine or the indoline moiety. The compounds, however, have not been disclosed to show any activity as inhibitors of the acetylcholinesterase enzyme.

When the double bond in formula (I) is present, the compounds represented are indoles.

Throughout the specification and appended claims, a given chemical formula or name shall encompass all geometrical and optical isomers and racemic mixtures where such isomers and mixtures exist.

Unless otherwise stated or indicated, the following definitions shall apply throughout the specification and the appended claims.

The term "$C_1$-$C_6$-alkyl" refers to a straight or branched chain hydrocarbon having from 1 to 6 carbon atoms, e.g., methyl, ethyl, propyl, isopropyl, n-butyl, neopentyl, n-hexyl, etc.

The term "alkoxy" refers to a monovalent substituent which consists of an alkyl group linked through an ether oxygen having its free valence bond from the ether oxygen; e.g., methoxy, ethoxy, propoxy, butoxy, etc.

The term "alkenyl" refers to acyclic hydrocarbons with one double bond of the general formula $C_nH_{2n}$, e.g., ethylene, butylene, etc.

The term "alkynyl" refers to acyclic hydrocarbons with one triple bond of the general formula $C_nH_{2n-2}$, e.g., acetylene, butyne, etc.

The term "halogen" refers to a member of the halogen family consisting of fluorine, chlorine, bromine and iodine.

The compounds of this invention are prepared in the following manner. The substituents $R_1$, X and Y are as defined above unless otherwise indicated.

Compound II of the formula

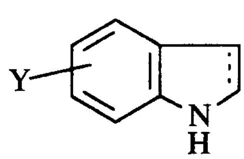

(II)

is reacted with a haloalkylpyridine hydrochloride of the formula

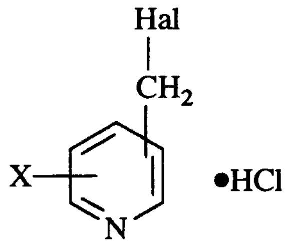

where Hal is halogen, to afford Compound III of the formula

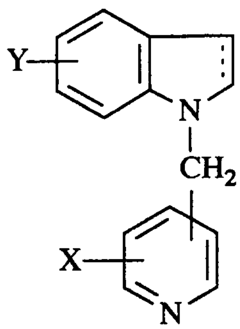

(III)

This reaction typically takes place in the presence of a base such as potassium hydroxide and a suitable solvent such as dimethylsulfoxide (DMSO) or dimethylformamide at ambient temperature to 50 ° C for 1 to 20 hours.

Compound III, where Y is phenylmethoxy, is hydrogenated in a routine manner, for instance, using a noble metal catalyst under a hydrogen atmosphere, to afford Compound IV of the formula

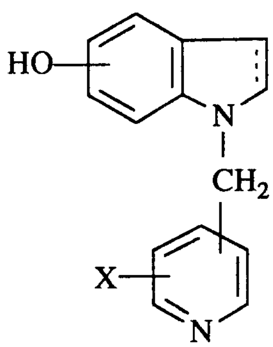

(IV)

The noble metal catalyst is selected from palladium or platinum on carbon. The reaction typically takes place at a temperature of about 20 ° C to 70 ° C for 1 to 20 hours.

Compound III may be reacted with n-butyllithium and a halide of the formula $R_1$-Hal, where $R_1$ is as previously defined and Hal is chlorine or bromine, in a suitable solvent to afford compound IIIa of the formula

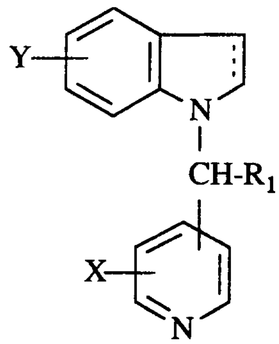

(IIIa)

Typically, this reaction takes place in tetrahydrofuran or ether at a temperature of about -80° to 0°C for 1 to 8 hours.

Compound IIIa, where Y is phenylmethoxy, is subsequently hydrogenated in a manner similar to that described above to afford Compound IV, where $R_1 \neq$ hydrogen.

The compounds of formula I of the present invention are useful in the treatment of various memory dysfunctions characterized by decreased cholinergic function, such as Alzheimer's disease. This utility is manifested by the ability of these compounds to inhibit the enzyme acetylcholinesterase and thereby increase acetylcholine levels in the brain.

**Cholinesterase Inhibition Assay**

Cholinesterases are found throughout the body, both in the brain and in serum. However, only brain acetylcholinesterase (AChE) distribution is correlated with central cholinergic innervation. This same innervation is suggested to be weakened in Alzheimer patients. We have determined in vitro inhibition of acetylcholinesterase activity in rat striatum according to the method described below.

**In Vitro Inhibition of Acetylcholinesterase Activity in Rat Striatum**

Acetylcholinesterase (AChE), which is sometimes called true or specific cholinesterase, is found in nerve cells, skeletal muscle, smooth muscle, various glands and red blood cells. AChE may be distinguished from other cholinesterases by substrate and inhibitor specificities and by regional distribution. Its distribution in the brain correlates with cholinergic innervation and subfractionation shows the highest level in nerve terminals.

It is generally accepted that the physiological role of AChE is the rapid hydrolysis and inactivation of acetylcholine. Inhibitors of AChE show marked cholinominetic effects in cholinergically-innervated effector organs and have been used therapeutically in the treatment of glaucoma, myasthenia gravis and paralytic ileus. However, recent studies have suggested that AChE inhibitors may also be beneficial in the treatment of Alzheimer's dementia.

The method described below was used in this invention for assaying anticholinesterase activity. This is a modification of the method of Ellman et al., Biochem. Pharmacol. 7, 88 (1961).

Procedure:

A. Reagents-
1. 0.05 M Phosphate buffer, pH 7.2
   (a) 6.85 g $NaH_2PO_4 \cdot H_2O$/100 ml distilled $H_2O$
   (b) 13.40 g $Na_2HPO_4 \cdot 7H_2O$/100 ml distilled $H_2O$
   (c) add (a) to (b) until pH reaches 7.2
   (d) Dilute 1:10
2. Substrate in buffer
   (a) 198 mg acetylthiocholine chloride (10 mM)
   (b) q.s. to 100 ml with 0.05 M phosphate buffer, pH 7.2 (reagent 1)
3. DTNB in buffer
   (a) 19.8 mg 5,5-dithiobisnitrobenzoic acid (DTNB) (0.5 mM)
   (b) q.s. to 100 ml with 0.05M phosphate buffer, pH 7.2 (reagent 1)

4. A 2mM stock solution of the test drug is made up in a suitable solvent and q.s. to volume with 0.5 mM DTNB (reagent 3). Drugs are serially diluted (1:10) such that the final concentration (in cuvette) is $10^{-4}$ M and screened for activity. If active, $IC_{50}$ values are determined from the inhibitory activity of subsequent concentrations.

B. Tissue Preparation -

Male Wistar rats are decapitated, brains rapidly removed, corpora striata dissected free, weighed and homogenized in 19 volumes (approximately 7 mg protein/ml) of 0.05 M phosphate buffer, pH 7.2, using a Potter-Elvehjem homogenizer. A 25 microliter aliquot of the homogenate is added to 1 ml of vehicle or various concentrations of the test drug and preincubated for 10 minutes at 37°C.

C. Assay

Enzyme activity is measured with the Beckman DU-50 spectrophotometer. This method can be used for $IC_{50}$ determinations and for measuring kinetic constants.

Instrument Settings

Kinetics Soft-Pac Module #598273 (10)
Program #6 Kindata:
Source - Vis
Wavelength - 412 nm
Sipper - none
Cuvettes - 2 ml cuvettes using auto 6-sampler
Blank - 1 for each substrate concentration
Interval time - 15 seconds (15 or 30 seconds for kinetics)
Total time - 5 minutes (5 or 10 minutes for kinetics)
Plot - yes
Span - autoscale
Slope - increasing
Results - yes (gives slope)
Factor - 1

Reagents are added to the blank and sample cuvettes as follows:

| | |
|---|---|
| Blank: | 0.8 ml Phosphate Buffer/DTNB |
| | 0.8 ml Buffer/Substrate |
| Control: | 0.8 ml Phosphate Buffer/DTNB/Enzyme |
| | 0.8 ml Phosphate Buffer/Substrate |
| Drug: | 0.8 ml Phosphate Buffer/DTNB/Drug/Enzyme |
| | 0.8 ml Phosphate Buffer/Substrate |

Blank values are determined for each run to control non-enzymatic hydrolysis of substrate and these values are automatically substracted by the kindata program available on kinetics soft-pac module. This program also calculates the rate of absorbance change for each cuvette.

For $IC_{50}$ Determinations:

Substrate concentration is 10 mM diluted 1:2 in assay yielding final concentration of 5 mM. DTNB concentration is 0.5 mM yielding 0.25 mM final concentration

$$\% \text{ Inhibition} = \frac{\text{slope control - slope drug}}{\text{slope control}} \times 100$$

Results of this assay for a representative compound of this invention and eseroline (reference) are presented in Table 1.

TABLE 1

| Inhibition of Brain Acetylcholinesterase Activity | |
|---|---|
| Compound | % Inhibition @ Dose |
| 1-(4-pyridinylmethyl)-1H-indol-5-ol | 24% @ 100 µM |
| Eseroline (Reference Compound) | 50% @ 6.4 µM |

This utility is further demonstrated by the ability of these compounds to restore cholinergically deficient memory in the Dark Avoidance Assay described below.

**Dark Avoidance Assay**

In this assay mice are tested for their ability to remember an unpleasant stimulus for a period of 24 hours. A mouse is placed in a chamber that contains a dark compartment; a strong incandescent light drives it to the dark compartment, where an electric shock is administered through metal plates on the floor. The animal is removed from the testing apparatus and tested again, 24 hours later, for the ability to remember the electric shock.

If scopolamine, an anticholinergic that is known to cause memory impairment, is administered before an animal's initial exposure to the test chamber, the animal re-enters the dark compartment shortly after being placed in the test chamber 24 hours later. This effect of scopolamine is blocked by an active test compound, resulting in a greater interval before re-entry into the dark compartment.

The results for an active compound are expressed as the percent of a group of animals in which the effect of scopolamine is blocked, as manifested by an increased interval between being placed in the test chamber and re-entering the dark compartment.

Results of this assay for a representative compound of this invention and those for 1-(4-pyridinylmethyl)-1H-indole, tacrine and pilocarpine (reference compounds) are presented in Table 2.

TABLE 2

| Compound | Dose (mg/kg of body weight, s.c.) | % of Animals with Scopolamine-Induced Memory Deficit Reversal |
|---|---|---|
| 1-[1-(4-pyridinyl)-butyl]-1H-indole | 0.3 | 20 |
| 1-(4-pyridinylmethyl)-1H-indole | 0.3<br>1.0 | 33<br>20 |
| Tacrine<br>Pilocarpine | 0.63<br>5.0 | 13<br>13 |

Effective quantities of the compounds of the present invention may be administered to a subject by any one of various methods, for example, orally as in capsules or tablets, parenterally in the form of sterile solutions or suspensions, and in some cases intravenously in the form of sterile solutions. The compounds of the present invention, while effective themselves, may be formulated and administered in the form of their pharmaceutically acceptable addition salts for purposes of stability, convenience of crystallization, increased solubility and the like.

Preferred pharmaceutically acceptable addition salts include salts of inorganic acids such as hydrochloric, hydrobromic, sulfuric, nitric, phosphoric and perchloric acids; as well as organic acids such as tartaric, citric, acetic, succinic, maleic, fumaric, and oxalic acids.

The active compounds of the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 75% of the weight of the unit. The amount of compound present in such composition is such that a suitable dosage will be obtained. Preferred compositions and preparations according to the present invention are prepared so that an oral dosage unit form contains between 1.0-300 mgs of active compound.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel™, corn starch and the like; a lubricant such as magnesium stearate or Sterotex®; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as fatty oil. Other dosage unit forms may contain other various materials which

modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of the aforesaid compound, but may be varied between 0.5 and about 30% of the weight thereof. The amount of active compound in such compositions is such that a suitable dosage will be obtained. Preferred compositions and preparations according to the present invention are prepared so that a parenteral dosage unit contains between 0.5 to 100 mgs of active compound.

The solutions or suspensions may also include the following components; a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

Examples of the compounds of the invention are listed below:

5-phenylmethoxy-1-(4-pyridinylmethyl)-1H-indole;
1-(4-pyridinylmethyl)-1H-indol-5-ol;
1-[1-(4-pyridinyl)butyl]-1H-indole;
5-phenylmethoxy-1-[1-(4-pyridinyl)butyl]-1H-indole;
1-[1-(4-pyridinyl)butyl]-1H-indol-5-ol;
1-[1-(4-pyridinyl)butyl]-5-methoxy-1H-indole;
1-[1-(3-methoxy-4-pyridinyl)butyl]-1H-indole;
1-[1-(3-fluoro-4-pyridinyl)butyl]-1H-indole;
1-[1-(3-fluoro-4-pyridinyl)butyl]-5-phenylmethoxy-1H-indole;
5-chloro-1-(4-pyridinylmethyl)-1H-indole;
5-methyl-1-[1-(4-pyridinyl)butyl]-1H-indole;
2,3-dihydro-1-[1-(4-pyridinyl)butyl]-1H-indole;
2,3-dihydro-5-phenylmethoxy-1-(4-pyridinylmethyl)-1H-indole;
2,3-dihydro-5-fluoro-1-[1-(4-pyridinyl)butyl]-1H-indole; and
2,3-dihydro-1-[1-(3-fluoro-4-pyridinyl)butyl]-5-methoxy-1H-indole.

The following examples are for illustrative purposes and are not to be construed as limiting the invention disclosed herein. All temperatures are given in degrees centigrade (°C) unless indicated otherwise.

## Example 1

### 5-Phenylmethoxy-1-(4-pyridinylmethyl)-1H-indole

To a solution of 5-phenylmethoxy-1H-indole (22.6 g) in 130 ml dimethylsulfoxide, was added milled potassium hydroxide (18 g) and the mixture was stirred at ambient temperature for two hours. The mixture was cooled with an ice bath, then 4-chloromethylpyridine hydrochloride (16.4 g) was added portionwise over a period of fifteen minutes. After stirring at ambient temperature for four hours, the mixture was poured into two liters ice water and stirred for ten minutes. The resultant precipitate was collected, washed with water and dissolved in ethyl acetate. The organic layer was washed with water and saturated sodium chloride and dried over anhydrous magnesium sulfate.

After filtering, the solution was evaporated to afford a solid, (~30 g) which was triturated with ether, collected and dried to give 27 g of a solid, m.p. 123-125°C. A sample of this material was eluted on a silica gel column with ethyl acetate/dichloromethane (1:2) via high pressure liquid chromatography (HPLC). The desired fractions were combined to yield 5-phenylmethoxy-1-(4-pyridinylmethyl)-1H-indole, as a solid, m.p. 124-5°C.

| Analysis: | | | |
|---|---|---|---|
| Calculated for $C_{21}H_{18}N_2O$: | 80.23%C | 5.77%H | 8.91%N |
| Found: | 79.92%C | 5.41%H | 8.79%N |

**Example 2**

**1-(4-Pyridinylmethyl)-1H-indol-5-ol**

To a suspension of 10% Pd/C (1.5 g) in 50 ml ethanol in a 500 ml Parr hydrogenation bottle, was added a suspension of 5-phenylmethoxy-1-(4-pyridinylmethyl)-1H-indole in 200 ml ethanol. The mixture was shaken at 50°C under 3.45 bar (50 psi) hydrogen for one hour, then cooled, filtered, and the filtrate evaporated to a solid, 10 g, dec. 178°C. A sample of this material was eluted on a silica gel column with 2% methanol/dichloromethane via HPLC. The desired fractions were combined and evaporated to yield 2.3 g of 1-(4-pyridinylmethyl)-1H-indol-5-ol, as a solid, m.p. 185-186°C.

| Analysis: | | | |
|---|---|---|---|
| Calculated for $C_{14}H_{12}N_2O$: | 74.98%C | 5.40%H | 12.49%N |
| Found: | 74.76%C | 5.46%H | 12.34%N |

Preparation - **Example 3**

**1-(4-Pyridinylmethyl)-1H-indole**

To KOH (34 g) in 200 ml of DMSO was added indole (20 g), portionwise, and this mixture was stirred for 90 minutes at room temperature. 4-Chloromethylpyridine hydrochloride (10 g) was added portionwise, and the mixture was stirred for four hours at room temperature. The mixture was then poured into water and extracted with ether four times. The organics were combined and washed with 2N HCl. The acidic aqueous phase was then basified with $NH_4OH$ and extracted with ether three times. The organics were combined and washed with NaCl and dried (anhy. $MgSO_4$). After filtering, the solvent was evaporated to yield an oil (14.5 g), which was eluted with 50% ethyl acetate/dichloromethane on a silica gel column via HPLC. The desired fractions were evaporated to yield 10.5 g of 1-(4-pyridinylmethyl)-1H-indole, as a solid, m.p. 65-68°C.

| Analysis: | | | |
|---|---|---|---|
| Calculated for $C_{14}H_{12}N_2$: | 80.74%C | 5.81%H | 13.45%N |
| Found: | 80.59%C | 5.87%H | 13.45%N |

**Example 4**

**1-[1-(4-Pyridinyl)butyl]-1H-indole**

To a solution of 1-(4-pyridinylmethyl)-1H-indole (4.0 g) in 100 ml of THF cooled to -78°C was added n-butyllithium (7.6 ml) dropwise, and the mixture was stirred for 45 minutes. 1-Bromopropane (2.34 g) was added dropwise, and the mixture was stirred for 3.5 hours, allowing the temperature to rise to 0°C. The mixture was quenched with water and extracted with ethyl acetate. The organic layer was washed with water and dried (sat. NaCl, anhy. $MgSO_4$). After filtering, the solvent was evaporated to yield an oil (3.8 g) which was eluted with 50% ethyl acetate/dichloromethane on a silica gel column via HPLC. The desired fractions were evaporated to yield 2.65 g of 1-[1-(4-pyridinyl)butyl]-1H-indole, as a solid, m.p. 91-94°C.

| Analysis: | | | |
|---|---|---|---|
| Calculated for $C_{17}H_{18}N_2$: | 81.56%C | 7.25%H | 11.19%N |
| Found: | 81.66%C | 7.32%H | 11.22%N |

**Example 5**

**5-Phenylmethoxy-1-[1-(4-pyridinyl)butyl]-1H-indole oxalate**

To a solution of 5-phenylmethoxy-1-(4-pyridinylmethyl)-1H-indole (11.4 g) in 120 ml tetrahydrofuran at -78 °C, was added n-butyllithium (2.5 M solution in hexane, 14.4 ml) and the solution was stirred at -78 °C for one hour. 1-Bromopropane (30 ml) was added to the solution and the mixture was allowed to warm to ambient temperature over a period of two hours. The mixture was poured into 300 ml water, stirred for five minutes and extracted twice with ethyl acetate. The organic layer was washed with water and dried (saturated NaCl solution, anhydrous $MgSO_4$). After filtering, the solvent was evaporated to an oil, (13.5 g) which was eluted on a silica gel column with ethyl acetate/dichloromethane (1:2) via HPLC. The desired fractions were combined and evaporated to give 8.2 g of an oil. A 1.0 g aliquot of this oil was dissolved in 10 ml methanol, and a solution of oxalic acid (0.3 g) in 5 ml methanol was added. The solution was diluted with 150 ml ether and the resultant precipitate was collected and dried to give 1.2 g of 5-phenylmethoxy-1-[1-(4-pyridinyl)butyl]-1H-indole oxalate, m.p. 148-149 °C.

| Analysis: | | | |
|---|---|---|---|
| Calculated for $C_{24}H_{24}N_2O \bullet C_2H_2O_4$: | 69.94%C | 5.87%H | 6.28%N |
| Found: | 70.09%C | 5.83%H | 6.22%N |

**Example 6**

**1-[1-(4-Pyridinyl)butyl]-1H-indol-5-ol**

To a suspension of 1.0 g of 10% Pd/C in 50 ml ethanol, was added a solution of 5-phenylmethoxy-1-[1-(4-pyridinyl)butyl]-1H-indole (7.2 g) in 200 ml ethanol. After shaking under under 3.45 bar (50 psi) hydrogen at 50 °C for one hour, the mixture was filtered, then evaporated to a solid, which was triturated with hexanes to give 5.0 g of 1-[1-(4-pyridinyl)butyl]-1H-indol-5-ol, m.p. 73-75 °C.

| Analysis: | | | |
|---|---|---|---|
| Calculated for $C_{17}H_{18}N_2O$: | 76.66%C | 6.81%H | 10.52%N |
| Found: | 76.23%C | 6.86%H | 10.13%N |

## Claims

**Claims for the following Contracting States : AT, BE, CH, DE, DK, FR, GB, IT, LI, LU, NL, SE**

1. A compound of the formula I

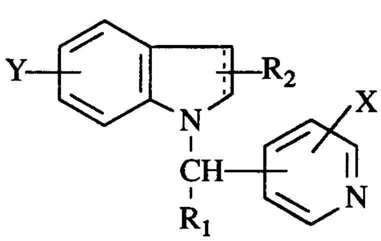

(I)

wherein

- $R_1$ is hydrogen, $C_1$-$C_6$-alkyl, phenyl-$C_1$-$C_6$-alkyl, $C_2$-$C_6$-alkenyl or $C_2$-$C_6$-alkynyl;
- $R_2$ is hydrogen;
- X is hydrogen, halogen, nitro, amino, $C_1$-$C_6$-alkyl, $C_1$-$C_6$-alkoxy or hydroxy;
- Y is hydrogen, $C_1$-$C_6$-alkyl, $C_1$-$C_6$-alkoxy, phenyl-$C_1$-$C_6$-alkoxy, hydroxy, halogen, nitro or amino;

the dotted line in formula I signifies an optional double bond; and wherein the phenyl group in each occurrence may be mono- or disubstituted with $C_1$-$C_6$-alkyl, $C_1$-$C_6$-alkoxy, halogen or trifluoromethyl, and with the proviso that at least one of the substituents $R_1$ and Y is not hydrogen; the pharmaceutically acceptable acid addition salts thereof, and, where applicable, the geometric and optical isomers and racemic mixtures thereof.

**2.** The compound as defined in claim 1 wherein Y is phenyl-$C_1$-$C_6$-alkoxy, wherein the phenyl may he substituted as defined in claim 1.

**3.** The compound as defined in claim 1 wherein Y is hydroxy.

**4.** The compound as defined in claim 1 wherein Y is hydrogen.

**5.** The compound as defined in claim 1 which is selected from the group consisting of 5-phenylmethoxy-1-(4-pyridinylmethyl)-1H-indole, 1-(4-pyridinylmethyl)-1H-indol-5-ol, 1-[1-(4-pyridinyl)butyl]-1H-indole, 5-phenylmethoxy-1-[1-(4-pyridinyl)butyl]-1H-indole and 1-[1-(4-pyridinyl)butyl]-1H-indol-5-ol.

**6.** A method of synthesizing a compound as defined in claim 1 which comprises reacting a compound of the formula

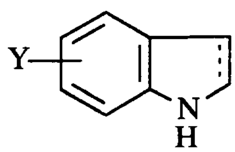

where Y is as defined in claim 1, with a haloalkylpyridine hydrochloride of the formula

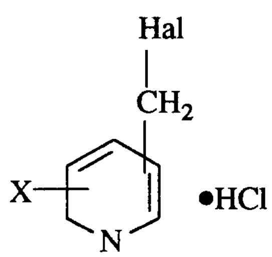

where Hal is halogen and X is as defined in claim 1,
in the presence of a base and suitable solvent, and optionally reacting a compound of the formula I, wherein $R_1$ is hydrogen, with n-butyllithium and a halide of the formula $R_1$-Hal, where Hal is chlorine or bromine and $R_1$ is as defined in claim 1, but is not hydrogen.

**7.** A pharmaceutical composition which comprises a compound as defined in claim 1 in an amount effective for alleviating a memory dysfunction characterized by a cholinergic deficit and a pharmaceutically acceptable carrier therefor.

**8.** Use of a compound of the formula I as defined in claim 1 for the preparation of the medicament having memory dysfunction alleviating activity.

**Claims for the following Contracting States : ES, GR**

1. A method of synthesizing a compound of the formula I

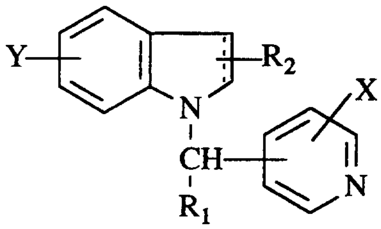

(I)

wherein

- $R_1$ is hydrogen, $C_1$-$C_6$-alkyl, phenyl-$C_1$-$C_6$-alkyl, $C_2$-$C_6$-alkenyl or $C_2$-$C_6$-alkynyl;
- $R_2$ is hydrogen;
- X is hydrogen, halogen, nitro, amino, $C_1$-$C_6$-alkyl, $C_1$-$C_6$-alkoxy or hydroxy;
- Y is hydrogen, $C_1$-$C_6$-alkyl, $C_1$-$C_6$-alkoxy, phenyl-$C_1$-$C_6$-alkoxy, hydroxy, halogen, nitro or amino;

the dotted line in formula I signifies an optional double bond; and wherein the phenyl group in each occurrence may be mono- or disubstituted with $C_1$-$C_6$-alkyl, $C_1$-$C_6$-alkoxy, halogen or trifluoromethyl, and with the proviso that at least one of the substituents $R_1$ and Y is not hydrogen; the pharmaceutically acceptable acid addition salts thereof, and, where applicable, the geometric and optical isomers and racemic mixtures thereof, which comprises
reacting a compound of the formula

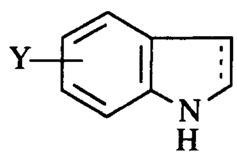

where Y is as defined above, with a haloalkylpyridine hydrochloride of the formula

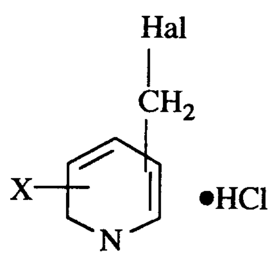

where Hal is halogen and X is as defined above,
in the presence of a base and suitable solvent, and optionally reacting a compound of the formula I, wherein $R_1$ is hydrogen, with n-butyllithium and a halide of the formula $R_1$-Hal, where Hal is chlorine or bromine and $R_1$ is as defined above, but is not hydrogen.

2. The method of claim 1 where the base is potassium hydroxide.

3. The method of claim 1 where the solvent is selected from dimethylsulfoxide and dimethylformamide.

4. The method of claim 1 wherein Y is phenyl-$C_1$-$C_6$-alkoxy, wherein the phenyl may be substituted as defined in claim 1.

5. The method of claim 1 wherein Y is hydroxy.

6. The method of claim 1 wherein a compound is prepared which is selected from the group consisting of 5-phenylmethoxy-1-(4-pyridinylmethyl)-1H-indole, 1-(4-pyridinylmethyl)-1H-indol-5-ol, 1-[1-(4-pyridinyl)-butyl]-1H-indole, 5-phenylmethoxy-1-[1-(4-pyridinyl)butyl]-1H-indole and 1-[1-(4-pyridinyl)butyl]-1H-indol-5-ol.

**Patentansprüche**

**Patentansprüche für folgende Vertragsstaaten : AT, BE, CH, DE, DK, FR, GB, IT, LI, LU, NL, SE**

1. Verbindung der Formel I

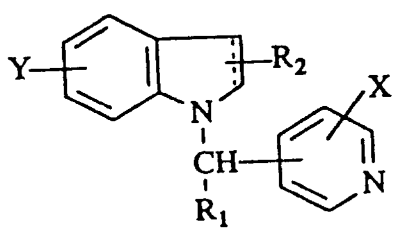

(I)

in welcher

$R^1$ für Wasserstoff, $C_1$-$C_6$-Alkyl, Phenyl-$C_1$-$C_6$-alkyl, $C_2$-$C_6$-Alkenyl oder $C_2$-$C_6$-Alkynyl steht;
$R_2$ Wasserstoff ist;
X Wasserstoff, Halogen, Nitro, Amino, $C_1$-$C_6$-Alkyl, $C_1$-$C_6$-Alkoxy oder Hydroxy bedeutet;
Y für Wasserstoff, $C_1$-$C_6$-Alkyl, $C_1$-$C_6$-Alkoxy, Phenyl-$C_1$-$C_6$-alkoxy, Hydroxy, Halogen, Nitro oder Amino steht;

die gestrichelte Linie in Formel I eine fakultative Doppelbindung darstellt;
und in welcher die Phenylgruppe bei jedem Auftreten einfach oder zweifach mit $C_1$-$C_6$-Alkyl, $C_1$-$C_6$-Alkoxy, Halogen oder Trifluormethyl substituiert sein kann, mit der Bedingung, daß mindestens einer der Substituenten $R_1$ und Y nicht Wasserstoff ist;
sowie ihrer pharmazeutisch verträglichen Säureadditionssalze und, gegebenenfalls, ihrer geometrischen und optischen Isomere und racemischen Gemische.

2. Verbindung gemäß Anspruch 1, in welcher Y für Phenyl-$C_1$-$C_6$-alkoxy steht, wobei das Phenyl wie in Anspruch 1 angegeben substituiert sein kann.

3. Verbindung gemäß Anspruch 1, in welcher Y für Hydroxy steht.

4. Verbindung gemäß Anspruch 1, in welcher Y für Wasserstoff steht.

5. Verbindung gemäß Anspruch 1, die aus der aus 5-Phenylmethoxy-1-(4-pyridinylmethyl)-1H-indol, 1-(4-Pyridinylmethyl)-1H-indol-5-ol, 1-[1-(4-Pyridinyl)butyl]-1H-indol, 5-Phenylmethoxy-1-[1-(4-pyridinyl)-butyl]-1H-indol und 1-[1-(4-Pyridinyl)butyl]-1H-indol-5-ol bestehenden Gruppe ausgewählt ist.

6. Verfahren zur Synthese einer Verbindung gemäß Anspruch 1, umfassend das Umsetzen einer Verbindung der Formel

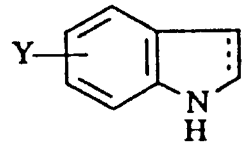

in welcher Y wie in Anspruch 1 angegeben definiert ist, mit einem Halogenalkylpyridinhydrochlorid der Formel

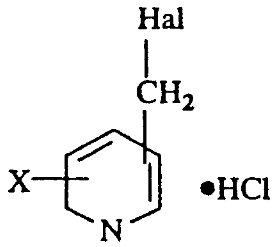

in welcher Hal für Halogen steht und X die in Anspruch 1 angewiesene Bedeutung zukommt, in Gegenwart einer Base und eines geeigneten Lösemittels, und wahlweise Umsetzen einer Verbindung der Formel I, in welcher $R_1$ Wasserstoff ist, mit n-Butyllithium und einem Halogenid der Formel $R_1$-Hal, in welcher Hal für Chlor oder Brom steht und $R_1$ wie in Anspruch 1 angegeben definiert ist, jedoch nicht Wasserstoff ist.

**7.** Pharmazeutische Zusammensetzung, die eine Verbindung gemäß Anspruch 1 in einer zur Erleichterung von Gedächtnisstörungen, die durch ein cholinergisches Defizit gekennzeichnet sind, wirksamen Menge und eine pharmazeutisch verträgliche Trägersubstanz dafür enthält.

**8.** Verwendung einer Verbindung der Formel I gemäß Anspruch 1 zur Herstellung des Arzneimittels mit Wirksamkeit zur Erleichterung von Gedächtnisstörungen.

**Patentansprüche für folgende Vertragsstaaten : ES, GR**

**1.** Verfahren zur Herstellung einer Verbindung der Formel I

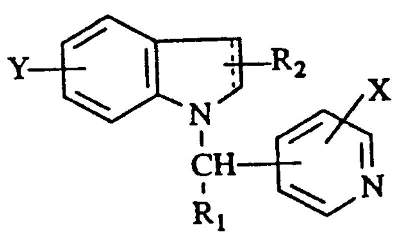

(I)

in welcher

- $R_1$ für Wasserstoff, $C_1$-$C_6$-Alkyl, Phenyl-$C_1$-$C_6$-alkyl, $C_2$-$C_6$-Alkenyl oder $C_2$-$C_6$-Alkynyl steht;
- $R_2$ Wasserstoff ist;
- X Wasserstoff, Halogen, Nitro, Amino, $C_1$-$C_6$-Alkyl, $C_1$-$C_6$-Alkoxy oder Hydroxy bedeutet;
- Y für Wasserstoff, $C_1$-$C_6$-Alkyl, $C_1$-$C_6$-Alkoxy, Phenyl-$C_1$-$C_6$-alkoxy, Hydroxy, Halogen, Nitro oder Amino steht;

die gestrichelte Linie in Formel I eine fakultative Doppelbindung darstellt;
und in welcher die Phenylgruppe bei jedem Auftreten einfach oder zweifach mit $C_1$-$C_6$-Alkyl, $C_1$-$C_6$-Alkoxy, Halogen oder Trifluormethyl substituiert sein kann, mit der Bedingung, daß mindestens einer der Substituenten $R_1$ und Y nicht Wasserstoff ist;
sowie ihrer pharmazeutisch verträglichen Säureadditionssalze und, gegebenenfalls, ihrer geometrischen und optischen Isomere und racemischen Gemische,
umfassend das Umsetzen einer Verbindung der Formel

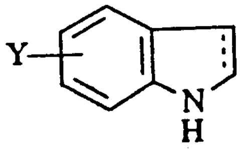

in welcher Y wie vorstehend angegeben definiert ist, mit einem Halogenalkylpyridinhydrochlorid der Formel

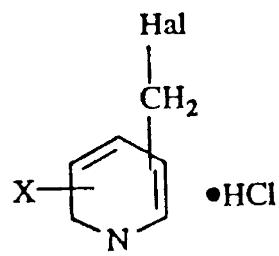

in welcher Hal für Halogen steht und X die vorstehend angewiesene Bedeutung zukommt, in Gegenwart einer Base und eines geeigneten Lösemittels, und wahlweise Umsetzen einer Verbindung der Formel I, in welcher $R_1$ Wasserstoff ist, mit n-Butyllithium und einem Halogenid der Formel $R_1$-Hal, in welcher Hal für Chlor oder Brom steht und $R_1$ wie vorstehend angegeben definiert ist, jedoch nicht Wasserstoff ist.

2. Verfahren gemäß Anspruch 1, bei dem die Base Kaliumhydroxid ist.

3. Verfahren gemaß Anspruch 1, bei dem das Lösemittel aus Dimethylsulfoxid und Dimethylformamid ausgewählt wird.

4. Verbindung gemäß Anspruch 1, in welcher Y für Phenyl-$C_1$-$C_6$-alkoxy steht, wobei das Phenyl wie in Anspruch 1 angegeben substituiert sein kann.

5. Verbindung gemäß Anspruch 1, in welcher Y für Hydroxy steht.

6. Verbindung gemäß Anspruch 1, die aus der aus 5-Phenylmethoxy-1-(4-pyridinylmethyl)-1H-indol, 1-(4-Pyridinylmethyl)-1H-indol-5-ol, 1-[1-(4-Pyridinyl)butyl]-1H-indol, 5-Phenylmethoxy-1-[1-(4-pyridinyl)-butyl]-1H-indol und 1-[1-(4-Pyridinyl)butyl]-1H-indol-5-ol bestehenden Gruppe ausgewählt ist.

**Revendications**

**Revendications pour les Etats contractants suivants : AT, BE, CH, DE, DK, FR, GB, IT, LI, LU, NL, SE**

1. Composé de formule I

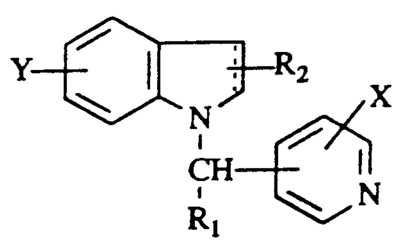

(I)

dans laquelle

- $R_1$ est un atome d'hydrogène ou un groupe alkyle en $C_1$-$C_6$, phényl-alkyle($C_1$-$C_6$), alcényle en $C_2$-$C_6$ ou alcynyle en $C_2$-$C_6$;
- $R_2$ est un atome d'hydrogène;
- X est un atome d'hydrogène ou d'halogène, ou un groupe nitro, amino, alkyle en $C_1$-$C_6$, alcoxy en $C_1$-$C_6$ ou hydroxy;
- Y est un atome d'hydrogène ou d'halogène, ou un groupe alkyle en $C_1$-$C_6$, alcoxy en $C_1$-$C_6$, phényl-alcoxy($C_1$-$C_6$), hydroxy, nitro ou amino;

le trait interrompu dans la formule I signifie une double liaison facultative, et le radical phényle en chaque occurrence pouvant être mono- ou disubstitué par un ou des atomes

d'halogène ou groupes alkyle en $C_1$-$C_6$, alcoxy en $C_1$-$C_6$ ou trifluorométhyle, étant entendu qu'au moins l'un des substituants $R_1$ et Y n'est pas un atome d'hydrogène;
sels d'addition avec des acides pharmaceutiquement acceptables de celui-ci, et lorsque cela est applicable, isomères géométriques et optiques, et mélanges racémiques, de celui-ci.

**2.** Composé selon la revendication 1, dans lequel Y est un groupe phényl-alcoxy($C_1$-$C_6$), le fragment phényle pouvant être substitué comme défini dans la revendication 1.

**3.** Composé selon la revendication 1, dans lequel Y est le groupe hydroxy.

**4.** Composé selon la revendication 1, dans lequel Y est un atome d'hydrogène.

**5.** Composé selon la revendication 1, qui est choisi parmi le 5-phénylméthoxy-1-(4-pyridinylméthyl)-1H-indole, le 1-(4-pyridinylméthyl)-1H-indol-5-ol, le 1-(4-pyridinyl)butyl]-1H-indole, le 5-phénylméthoxy-1-[1-(4-pyridinyl)butyl]-1H-indole et le 1-[1-(4-pyridinyl)butyl]-1H-indol-5-ol.

**6.** Procédé de synthèse d'un composé selon la revendication 1, comprenant la mise en réaction d'un composé de formule

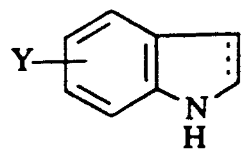

dans laquelle Y est tel que défini dans la revendication 1, avec un chlorhydrate d'halogénoalkylpyridine de formule

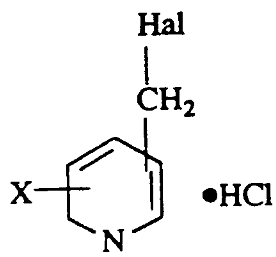

dans laquelle Hal est un atome d'halogène et X est tel que défini dans la revendication 1,
en présence d'une base et d'un solvant approprié, et éventuellement la mise en réaction d'un composé de formule I, dans laquelle $R_1$ est un atome d'hydrogène, avec du n-butyllithium et un halogénure de formule $R_1$-Hal, dans laquelle Hal est un atome de chlore ou de brome et $R_1$ est tel que défini dans la revendication 1, mais n'est pas un atome d'hydrogène.

**7.** Composition pharmaceutique comprenant un composé tel que défini dans la revendication 1, en une quantité efficace pour pallier un dysfonctionnement de la mémoire caractérisé par une déficience cholinergique, et un véhicule pharmaceutiquement acceptable pour celui-ci.

**8.** Utilisation d'un composé de formule I tel que défini dans la revendication I, pour la fabrication d'un médicament destiné à pallier un dysfonctionnement de la mémoire.

**Revendications pour les Etats contractants suivants : ES, GR**

1. Procédé de synthèse d'un composé de formule I

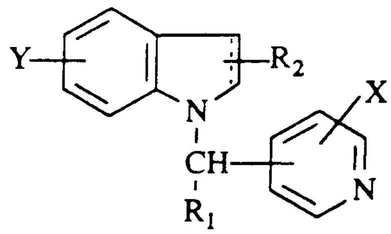

(I)

dans laquelle

- $R_1$ est un atome d'hydrogène ou un groupe alkyle en $C_1$-$C_6$, phényl-alkyle($C_1$-$C_6$), alcényle en $C_2$-$C_6$ ou alcynyle en $C_2$-$C_6$;
- $R_2$ est un atome d'hydrogène;
- X est un atome d'hydrogène ou d'halogène, ou un groupe nitro, amino, alkyle en $C_1$-$C_6$, alcoxy en $C_1$-$C_6$ ou hydroxy;
- Y est un atome d'hydrogène ou d'halogène, ou un groupe alkyle en $C_1$-$C_6$, alcoxy en $C_1$-$C_6$, phényl-alcoxy($C_1$-$C_6$), hydroxy, nitro ou amino;

le trait interrompu dans la formule I signifie une double liaison facultative, et le radical phényle en chaque occurrence pouvant être mono- ou disubstitué par un ou des atomes d'halogène ou groupes alkyle en $C_1$-$C_6$, alcoxy en $C_1$-$C_6$ ou trifluorométhyle, étant entendu qu'au moins l'un des substituants $R_1$ et Y n'est pas un atome d'hydrogène;
de ses sels d'addition avec des acides pharmaceutiquement acceptables, et lorsque cela est applicable, de ses isomères géométriques et optiques, et mélanges racémiques,
comprenant
la mise en réaction d'un composé de formule

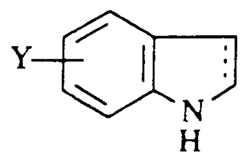

dans laquelle Y est tel que défini plus haut, avec un chlorhydrate d'halogénoalkylpyridine de formule

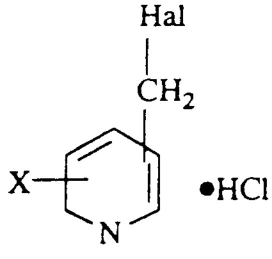

dans laquelle Hal est un atome d'halogène et X est tel que défini plus haut,
en présence d'une base et d'un solvant approprié, et éventuellement la mise en réaction d'un composé de formule I, dans laquelle $R_1$ est un atome d'hydrogène, avec du n-butyllithium et un halogénure de formule $R_1$-Hal, dans laquelle Hal est un atome de chlore ou de brome et $R_1$ est tel que défini plus haut, mais n'est pas un atome d'hydrogène.

2. Procédé selon la revendication 1, dans lequel la base est l'hydroxyde de potassium.

3. Procédé selon la revendication 1, dans lequel le solvant est choisi parmi le diméthylsulfoxyde et le diméthylformamide.

4. Procédé selon la revendication 1, dans lequel Y est un groupe phényl-alcoxy($C_1$-$C_6$), le fragment phényle pouvant être substitué comme défini dans la revendication 1.

5. Procédé selon la revendication 1, dans lequel Y est le groupe hydroxy.

6. Procédé selon la revendication 1, dans lequel on prépare un composé qui est choisi parmi le 5-phénylméthoxy-1-(4-pyridinylméthyl)-1H-indole, le 1-(4-pyridinylméthyl)-1H-indol-5-ol, le 1-(4-pyridinyl)-butyl]-1H-indole, le 5-phénylméthoxy-1-[1-(4-pyridinyl)butyl]-1H-indole et le 1-[1-(4-pyridinyl)butyl]-1H-indol-5-ol.